# EUROPEAN PATENT SPECIFICATION

(11) **EP 1 259 249 B1**
(45) Date of publication and mention of the grant of the patent: **15.07.2009**
(21) Application number: 01913177.0
(22) Date of filing: 01.03.2001
(51) Int. Cl.: G01N 33/569, C07K 16/12, A61K 39/02, C07K 1/22, C12M 1/34, C12Q 1/04, G01N 33/53, G01N 33/543, G01N 33/553

(54) **METHOD FOR DETECTING THE PRESENCE OF TARGET BACTERIA OR A TARGET COMPONENT CARBOHYDRATE ANTIGEN THEREOF**
METHODE ZUM NACHWEIS VON (ZIEL)BAKTERIEN ODER KOHLENHYDRAT-ANTIGENEN DIESER (ZIEL)BAKTERIEN
PROCEDE PERMETTANT DE DETECTER LA PRESENCE DE BACTERIES CIBLES OU D'UN ANTIGENE CARBOHYDRATE CIBLE DE CES BACTERIES

(30) Priority: 01.03.2000 US 518165
(43) Date of publication of application: 27.11.2002
(73) Proprietor: Binax, Inc., Portland, ME 04103 (US)
(72) Inventor: MOORE, Norman, James, North Berwick, ME 03906 (US); FENT, Mary, Kathleen, Cumberland Center, ME 04021 (US); KOULCHIN, Vladimir, Andrei, Portland, ME 04101 (US); MOLOKOVA, Elena, Valentin, Portland, ME 04101 (US)
(74) Representative: Becker Kurig Straus
(86) International application number: PCT/US2001/006495
(87) International publication number: WO 2001/064237

(56) References cited:
- WO-A-00/10584
- WO-A-98/55871
- US-A- 4 206 094
- US-A- 4 411 832
- US-A- 5 415 994
- US-A- 5 843 463
- US-A- 6 018 019
- MOLOKOVA E ET AL: "C-21. THE DEVELOPMENT OF A RAPID IMMUNOCHROMATOGRAPHIC TEST (ICT) FOR THE DETECTION OF STREPTOCOCCUS PNEUMONIAE IN URINE" ABSTRACTS OF THE GENERAL MEETING OF THE AMERICAN SOCIETY FOR MICROBIOLOGY, THE SOCIETY, WASHINGTON, DC, US, vol. 99, 31 May 1999 (1999-05-31), page 109 XP001013628 ISSN: 1060-2011
- MORE N ET AL: "Development of an immunochromatographic (ICT) assay for the detection of Legionella pneumophila" ABSTRACTS OF THE GENERAL MEETING OF THE AMERICAN SOCIETY FOR, vol. 98, 1998, page 203 XP009024507 98th General Meeting of the American Society for Microbiology;Atlanta, Georgia, USA; May 17-21, 1998, 1998 ISSN: 1060-2011
- LINDBERG A A: "Polyosides (encapsulated bacteria)" COMPTES RENDUS DES SEANCES DE L'ACADEMIE DES SCIENCES. SERIE III: SCIENCES DE LA VIE, ELSEVIER, AMSTERDAM, NL, vol. 322, no. 11, November 1999 (1999-11), pages 925-932, XP004270330 ISSN: 0764-4469
- POXTON I R: "Antibodies to lipopolysaccharide" JOURNAL OF IMMUNOLOGICAL METHODS, ELSEVIER SCIENCE PUBLISHERS B.V.,AMSTERDAM, NL, vol. 186, no. 1, 12 October 1995 (1995-10-12), pages 1-15, XP004021134 ISSN: 0022-1759

## Description

### INTRODUCTION TO THE PRESENT INVENTION

The present invention relates to achieving rapid and accurate diagnoses, of high sensitivity and specificity, of bacterial infections caused by bacteria of the genus Haemophilus influenzae characterized by the possession of carbohydrate antigens. In particular, the invention involves the initial purification of such carbohydrate antigens to an essentially protein-free state, followed by utilization of each so-purified carbohydrate antigen to affinity purify raw polyvalent antibodies to said antigen and the utilization of the said so-purified antibodies in diagnostic tests of high accuracy, specificity and sensitivity for detecting the presence of the original bacterium.

The invention relates to Haemophilus influenzae possessing carbohydrate antigens, which bacteria are negative to Gram's stain. The purified antibodies are of at least the same order of specificity and sensitivity as commercially available monoclonal antibodies and are easier to produce and to work with than many such monoclonal antibodies. They offer wide opportunities for rapid diagnostic tests, *e.g.*, via ICT immunoassays, to identify bacteria that have heretofore been difficult to identify rapidly and accurately, whereby diagnoses of bacterial diseases they caused were often arrived at slowly and difficultly, using cumbersome methodology.

### BACKGROUND OF THIS INVENTION

Gram-negative bacteria are known to have in common the possession of at least one lipo-polysaccharide or other lipo-polycarbohydrate antigen, while Gram-positive bacteria are known to possess the common characteristic of having at least one carbohydrate antigen that is a lipo-teichoic acid or teichoic acid or a derivative of either. Some of both the Gram-positive and Gram-negative bacteria also possess carbohydrate antigens that are capsular - *i.e.*, these antigens are each enclosed in a heavy capsular layer in their native state. This capsular layer constitutes a slime-like substance that surrounds the bacterial cell wall of most bacteria.

U.S. Application Serial No. 09/139,720, describes the purification to an essentially protein-free state of lipo-carbohydrate antigens of bacteria of the *Legionella* species, all of which are Gram-positive. Emphasis is placed therein on purifying carbohydrate antigens of *Legionella pneumophila* serotypes, including without limitation, the O-polysaccharide antigen of *L. pneumophila* serotype 1, the purification of which to an essentially protein-free state, is described in detail.

The application shows that when the essentially protein-free O-carbohydrate antigen of *L. pneumophila* serotype 1 (which serotype is known to be the causative organism for some 70 percent of the *Legionella*-caused pneumonia-like illnesses that occur), is coupled (through a spacer molecule) to an affinity column as described and raw polyclonal antibodies to the unpurified antigen are passed over the column as described, the resulting purified antibodies are highly antigen-specific and will readily identify the same antigen when it is present in bodily fluids taken from patients with disease caused by *Legionella pneumophila* serotype 1. Urine is shown to be a preferred bodily fluid for this diagnostic purpose because:
(1) The *L. pneumophila* serotype 1 antigen appears in urine early in the disease state and persists for some days even after appropriate therapeutic treatment is initiated;
(2) The collection of the test sample is non-invasive and simple, causing a minimum of patient disruption as well as requiring no specially trained personnel or specially designed instrumentation; and
(3) Samples from, *e.g.,* sputum may give false negative or false positive results due to difficulties in obtaining or culturing the sample, possible presence of colonies of bacteria in the patient's nose or throat that are chronically present and were not causative of disease, and other similar difficulties.

The *L. pneumophila* serotype 1 bacterium present in urine is dead and has at least in part had its cell wall broken down as it is passed through the kidneys; hence the antigen is in a state readily accessible to the antigen-specific antibodies deposited in two areas on the ICT test strip.

The efficacy of the antigen-specific antibodies described in U.S. Application Serial No. 09/139,720 in identifying whole, and to some extent, living bacteria in aqueous media constituting environmental samples is further shown in the continuation-in-part Application Serial No. 09/458,988, wherein an enzyme immunoassay of high specificity and sensitivity is described. This assay is based on use as the detecting agent for the antigen, of antigen-specific antibodies obtained by purifying raw polyclonal antibodies as described in detail in the parent application. The sensitivity and specificity of the so-purified antibodies is in part illustrated by the short times within which the enzyme immunoassay produced informative results, as well as by the small concentration (0.05 µg per test) of antigen-specific antibodies that gave equally informative time results with a longer incubation time (1 hour).

U.S. Application Serial No. 09/397,110, describes the purification to an essentially protein-free state of the C-polysaccharide cell wall antigen present in the pneumococcal cell wall of all *S. pneumoniae* serotypes. This antigen is a phosphocholine-containing polysaccharide derived from teichoic acid. The *Streptococcus pneumoniae* strain of bacteria are all Gram-stain positive.

The essentially protein-free antigen (which contains less than about 10 percent by weight thereof) is covalently coupled to a spacer molecule which is in turn covalently coupled to an affinity column and the thus-prepared column is then used to purify raw polyclonal antibodies to *S. pneumonia.* The resulting antigen-specific purified antibodies showed high sensitivity and specificity in an ICT test for identifying *S. pneumoniae* in bodily fluids, including urine in particular.

Numerous and varied efforts have been made in the past to use raw polyvalent antibodies to carbohydrate antigens, or monoclonal antibodies to such antigens, of various infectious Gram-negative or Gram-positive bacteria believed to be responsible for diseases of the lower respiratory tract in diverse tests, including ELISA, counter-immunoelectrophoresis and/or latex agglutination tests for the presence of the specific bacterium sought. While some of the tests have been useful in some cases, none of them has so far gained sufficient clinical acceptance of reliability to be used independently of cell culture tests. The drawbacks of cell culture tests and their tenuous reliability have been extensively documented in the art and are discussed in parent applications 09/139,720 and 09/397,110.

Molokova et al. ("C-21. The development of a rapid immunochromatographic test (ICT) for the detection of Streptococcus pneumoniae in urine", Abstracts of the general meeting of the American Society for Microbiology, The Society, Washington, DC, Vol. 99, May 31, 1999, p. 109) reports the development of a rapid test for the detection of *S. pneumoniae.* Said test may be in the lateral flow membrane format and relies on C-polysaccharide specific affinity purified antibodies.

More et al. ("Development of an immunochromatographic (ICT) assay for the detection of Legionella pneumophila", Abstracts of the general meeting of the American Society for Microbiology, 98th meeting, Atlanta, GA, May 17-21, 1998) provides information about the development of an immunodiagnostic assay for screening *L. pneumophila* serogroup 1 in clinical and environmental specimen.

WO 98/55871 relates to a method for detecting an infection with enterohemorrhagic *E. coli.* For this purpose, anti-lipopolysaccharide antibodies in a sample are reacted with enterohemorrhagic *E. coli* lipo-polysaccharides immobilized on carrier particles. The infection is then detected via spectroscopic steps by determining the extent of agglutination.

WO 00/16803 relates to a specific and sensitive immunochromatographic ("ICT") assay, performable within about 15 minutes, for the detection of *Streptococcus pneumoniae* in a bodily fluid. In the assay antibodies to the C-polysaccharide antigen of *S. pneumoniae* raised in rabbits are affinity purified with isolated and purified C-polysaccharide antigen having less than about 10% protein content. These affinity purified antibodies are then conjugated to an agent which produces a color reaction upon the formation of a sandwich with *S. pneumoniae* C-polysaccharide antigen from a test sample and additional affinity purified C-polysaccharide antibody immobilized upon a nitrocellulose matrix.

To gain U.S. Food and Drug Administration ("FDA") approval of the *L. pneumophila* serogroup 1 ICT test first described in parent Application Serial No.. 09/139,720 and the *S. pneumoniae* ICT test that is the subject of parent Application Serial No. 09/397,110 and its parent application, Serial No. 09/156,786, it was necessary for the assignee of these applications, Binax, Inc., to conduct extensive clinical tests on each. Many of these clinical tests are described in the two parent applications. One of the important points about the clinical tests is that FDA regulations require extensive clinical testing of diagnostic tests only in instances where the diagnostic test is recognized to represent a substantial scientific and technical departure from tests that are already known and in commercial use. The sensitivity and specificity of each of these two tests is believed to be much higher than the numbers shown in the parent applications indicate. The reason is that the numbers shown are based on comparison of these clinical test results with parallel results obtained on the same clinical samples with other earlier available assay procedures or identification techniques (such as cell culture tests), which prior available tests were known to be tenuously reliable even when they were believed to be the best available identification methods for detecting the involved bacteria or their antigenic components.

In short, this invention presents the opportunity for providing a highly specific and sensitive, rapid diagnostic test for the detection of a target carbohydrate antigen component of Haemophilus influenzae.

### BRIEF DESCRIPTION OF THE INVENTION

The invention pertains to an immunochromatographic (ICT) assay for the detection of a target carbohydrate antigen component of *Haemophilus influenzae,* which comprises the steps of:
(a) contacting a sample of a fluid suspected of containing said target bacteria or their target carbohydrate antigen component with an immunochromatographic (ICT) device comprising a strip of a bibulous material, which strip has
   (i) a zone in which has been embedded a conjugate of: (1) a labeling agent that displays a visible color change upon reaction of antibodies with their corresponding antigenic binding partner, and (2) purified antigen-specific antibodies to the target carbohydrate antigen component, said antibodies having been purified by passage over a chromatographic affinity column to which is conjugated through a spacer molecule the essentially protein-free purified target carbohydrate antigen component;
   (ii) a second zone having bound thereto the same purified antigen specific antibodies in unconjugated form, which zone is equipped with a window for viewing color changes;
(b) allowing said sample to flow laterally along said test strip to said first zone;
(c) allowing said sample, together with said conjugate of affinity purified antibodies and label, to flow laterally along said test strip to said second zone; and
(d) within approximately 15 to 20 minutes from the commencement of step (a), observing through said window whether a line of color has appeared in said second zone, thereby indicating the presence in the sample of *Haemophilus influenzae,* or both, or whether no line of color has so appeared indicating the absence of *Haemophilus influenzae.*

In a preferred embodiment the target carbohydrate antigen component is a capsular carbohydrate antigen of *Haemophilus influenzae* type b bacteria, and the labeling agent is finely divided metallic gold.

In another preferred embodiment the antigen-specific antibodies are present in a concentration of between 7.7 nanograms/mm² of surface area and 385 nanograms/mm² of surface area at each site of a test device at which antigen : antibody reaction is to occur.

A ICT procedure for detecting the capsular polysaccharide antigen of *H. influenzae* type b is described herein in detail.

Heretofore it has not been recognized that the lipo-polycarbohydrate antigens typically found in Gram-negative bacteria, may all be detected by a rapid, highly specific and sensitive immunoassay of the ICT type which employs antigen-specific antibodies as the detecting agent, which antigen-specific antibodies are obtained according to the schema for purifying raw polyclonal antibodies to carbohydrate antigens that is set forth in the second paragraph of this section. The fact that raw polyvalent antibodies to bacterial carbohydrate antigens may be rendered highly antigen-specific and sensitive by subjecting them to affinity purification with a purified target bacterial carbohydrate antigen that is essentially protein-free likewise has not been appreciated heretofore. Likewise, the fact that carbohydrate antigens from both Gram-negative and Gram-positive bacteria and/or from the capsular layer surrounding both types of bacteria can all be purified and used to affinity purify antibodies to such antigens to yield antigen-specific antibodies has not been heretofore recognized, nor has it been appreciated that bacterial carbohydrate antigens can be detected rapidly with high accuracy, sensitivity and specificity using such antigen-specific antibodies as a detecting agent.

### BRIEF DESCRIPTION OF THE DRAWING

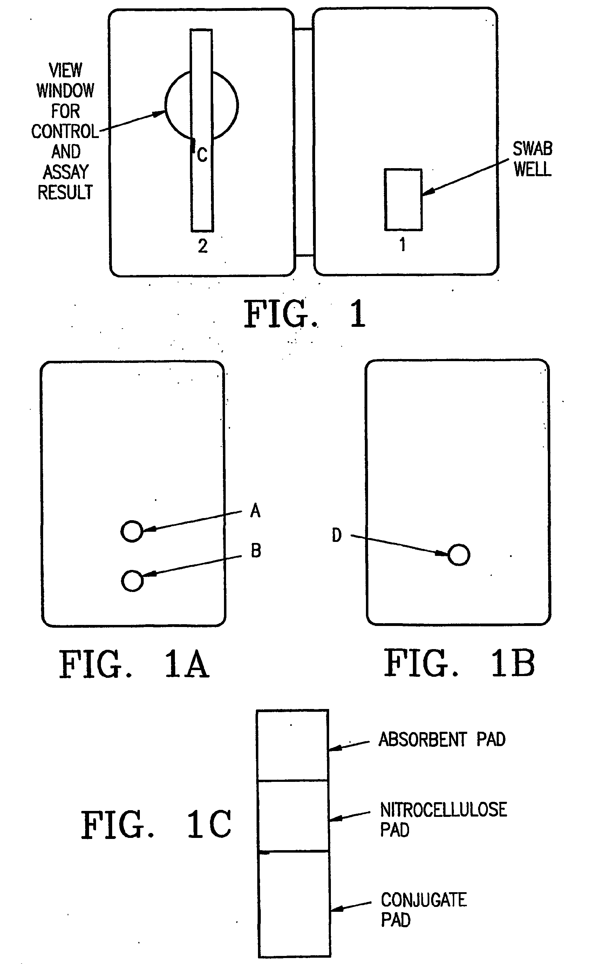

Figure 1 and its related Figures 1A, 1B and 1C depict a typical ICT device of the type preferred in the performance of an assay for a bacterial carbohydrate antigen in accordance with this invention.

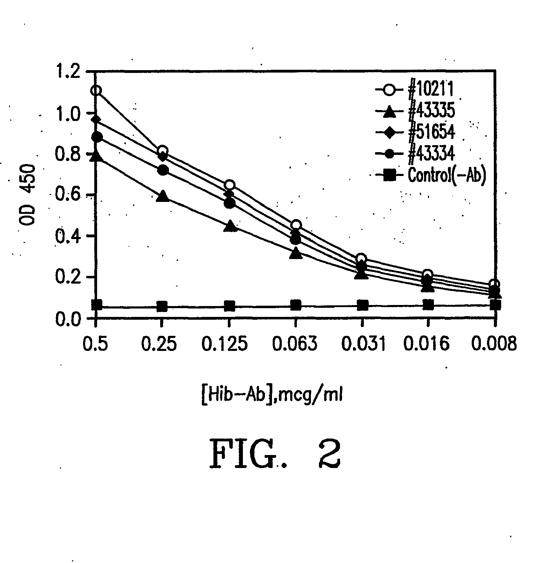

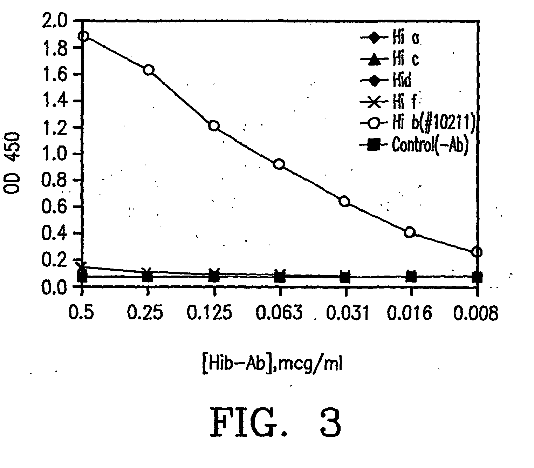

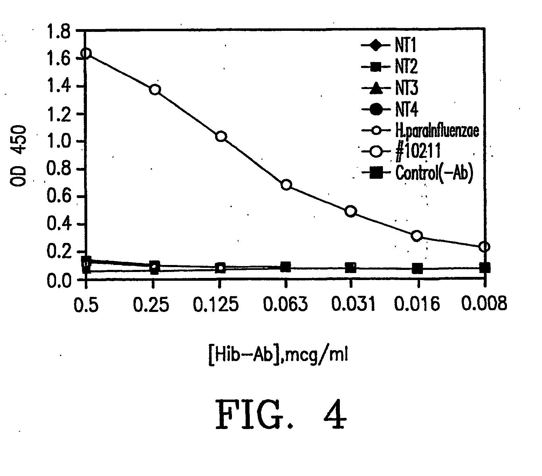

Figures 2, 3 and 4 are graphs showing, in Figure 2, the ability of antigen-specific purified antibodies of this invention to detect other serotypes of *H. influenzae* type b than the one to which the antibodies were raised. In Figures 3 and 4, the graphs reflect that the purified antigen-specific antibodies of *H. influenzae* type b were not cross-reactive with antigens of *H. influenzae* types a, c, d or f (Fig. 3) or with any of nontypical *H. influenzae* NT1, NT2, NT3 or NT4 or with *H. para-influenzae.*

### DETAILED DESCRIPTION OF THE INVENTION

The present invention represents an exceptional advance in methods for detecting bacterial infection by Haemophilus influenzae.

Of particular importance is the opportunity that this invention affords for rapid diagnosis and rapid introduction of appropriate therapy in situations where a particular bacterially-caused disease appears to be epidemic within a group - whether a small, confined group, *e.g.*, in a school or geriatric center, or a widespread population as, *e.g.*, a town, a city or a larger region.

Broadly speaking, the preferred immunochromatographic ("ICT") assay of this invention may be designed and configured to be run on any known disposable ICT device disclosed in the art. Preferably it is designed to be conducted, and is conducted, using an ICT device of the type disclosed in co-pending U.S. Patent Application Serial No. 07/706,639 of Howard Chandler, or one of its continuation-in-part applications, all of which are assigned to Smith-Kline Diagnostics, Inc. but are exclusively licensed to Binax, Inc. (which is entitled to assignment of this application), in a wide area of use fields that includes diagnoses of human respiratory system diseases.

The preferred device is suitably impregnated in one region thereof with affinity purified, highly antigen specific antibodies to the target carbohydrate antigen of Haemophilus influenzae suspected of causing the disease. Labeled antigen-specific antibodies are applied to another area of the device. The test sample suspected of containing the bacterium is contacted first with the labeled antigen-specific antibodies, which then flow with the sample to the device area containing unlabeled bound antigen-specific antibodies, whereupon if the target antigen indigenous to the suspected bacterium is present in the sample, the labeled antibody:target carbohydrate antigen conjugate already formed binds upon contact to the immobilized unlabeled antigen-specific antibodies, whereupon a visible color reaction is produced. The label may be any substance known in the art to produce visible color upon the reaction of a labeled antibody:antigen complex with bound unlabeled antibodies. Such labels include various finely divided metallics, various organic molecules, and various molecular combinations such as enzyme combinations with another color-producing molecule. In this invention, colloidal gold particles constitute the preferred label.

It is of major importance in designing the preferred test device, that the concentration of antigen-specific antibody present at each of the two sites of the test device where reaction occurs be sufficient to insure that antigen present in the test sample will be captured by the labeled antigen-specific antibodies as the test sample contacts them and that labeled antigen-specific antibody:antigen conjugate will be readily captured and held by the bound antibodies at the sample capture line. Experimental work undertaken in connection with this invention has shown that active antigen-specific antibody to the target carbohydrate antigen must be present at each site of a test device at which antigen:antibody reaction is to occur in a concentration of between 7.7 nanograms/sq. mm. of surface area and 385 nanograms/sq. mm. of surface area. If antigen-specific antibody concentrations lower than 7.7 nanograms/sq. mm. are present at a site where reaction is intended to occur, false negative results are likely to occur.

As is known in the art, infectious bacteria frequently have multiple antigenic components. For example, *S. pneumoniae* is known to have a capsular antigen in addition to the polysaccharide cell wall antigen which is the target of the assay described in parent applications Serial Nos. 09/156,486 and 09/397,110. The latter antigen was selected as the target antigen for the now-FDA-approved test which is described in these applications because that antigen is present in all known serotypes of *S. pneumoniae* and its relatively minor cross-reactivity as described in the herein incorporated application Serial No. 09/397,110 is of a nature that allows ready clinical differentiation of *S. pneumoniae*-caused infection from other infections. It is noted that previous published attempts to detect *S. pneumoniae* in bodily fluids have at best yielded systems having sensitivity and specificity in the 60-70 percent range with both polyclonal and monoclonal antibodies - a range unacceptable for reliable diagnostic purposes.

Among the mammalian fluids in which target carbohydrate antigens have been shown to be successfully detected in ongoing clinical work with the respective described and FDA-approved ICT tests for *L. pneumophila* serogroup 1 and *S. pneumoniae* are, in addition to the preferred urine, sputum, naso-pharyngeal exudates, middle ear fluid and cerebrospinal fluid. Other fluids in which these tests detect carbohydrate target antigens, when present, include blood and bronchial fluid.

Selection of the target carbohydrate antigen for any particular bacterium is necessarily based upon considerations of that antigen's cross-reactivity characteristics, whether it is known to be present in all or most serotypes of a bacterial strain, whether if peculiar to a particular serotype of a strain, that serotype is known to be the most common source of disease caused by the bacterium and like questions.

The polyclonal antibodies to be purified are raised by conventional methods, by injecting an animal, *e.g.*, a rabbit or goat, with the crude target antigen of the intended assay. Preferably the antigen preparation is subjected to heat killing of cells before injecting the animal. After an appropriate lapse of time, the animal is bled to obtain serum containing the desired antibodies, followed by purification of the latter. This serum may go through an intermediate purification step, *e.g.*, with ammonium sulfate or an ion exchange resin to produce an IgG cut or may be purified directly.

For purposes of the affinity purification, the same crude carbohydrate target antigen used to immunize the animal is grown in culture and then suitably purified to an essentially protein-free state. As used herein the expression "essentially protein-free state" means a state containing not more than - and preferably less than - about 10 percent (wt./wt.) of protein.

After the antigen is purified to the essentially protein-free state, it is coupled to a spacer molecule by covalent binding. Examples of suitable spacer molecules include hydrazine, bovine serum albumen ("BSA"), the conjugate of BSA and hydrazine and like molecules that are capable of covalently bonding to purified carbohydrate antigens at one end while retaining another reactive end that is capable of bonding covalently to an affinity gel.

The purified carbohydrate antigen:spacer molecule conjugate is next conjugated to an affinity gel and the gel is used to purify the raw polyvalent antibodies in serum obtained by bleeding the previously immunized animal, or an IgG cut thereof. The raw antibodies (or their IgG cut) are multiply applied to the affinity gel and are eluted from it as purified, highly antigen-specific antibodies.

The following examples illustrate the mode of affinity purification of antibodies to *Haemophilus influenzae* type b, including the preliminary separation and purification of the capsular carbohydrate antigen used in that purification. Many methods for effecting these separation and purification steps are known in the literature and may be substituted for those herein described so long as the purified antigen obtained is essentially protein-free.

### Example 1 - Culture Conditions for Culturing the Target Carbohydrate Antigen

*Haemophilus influenzae* type b (ATCC #10211) was grown in supplemented Mueller Hinton broth at 37° C. with 5 percent CO₂ for 24 hours without agitation.

The broth composition, per liter, was:

| | |
|---|---|
| Acid hydrolyzate of casein | 17.5 g. |
| Beef heart extract | 3.0 g. |
| Starch | 1.5 g. |

Supplements as follows were also present:

| | |
|---|---|
| Hematin | 15 mg./mL |
| NAD (nicotine adenine dinucleotide) | 15 mg./mL |
| Yeast extract | 5 mg./mL |

The pH of this mixture was 7.3 ± 0.1 as measured at 25° C.

### Example 2 - Purification of Carbohydrate Antigen

After 24 hours, 1.82 g. of cetyltrimethylammonium bromide CAS #57-09-0 was dissolved in 30 mL of distilled water and the solution was added to 500 mL of broth supernatant to yield a final concentration of 0.01 M cetyltrimethylammonium bromide. The mixture was incubated in an ice bath with stirring for one hour and left at 4° C. overnight.

The mixture from Example 1 was centrifuged at 12,000 rpm and 4° C. for 20 minutes to yield a pellet and a supernatant. Both were collected and treated, respectively, as follows:
(1) The pellet was resuspended in 0.5 M NaCl with sonication and was then dropwise precipitated at 4° C. in ten times the resuspension volume of ethanol. The resulting solution was stored overnight at 4° C. to allow precipitation.
   The solution was then centrifuged at 12,000 rpm for 20 minutes. The pellet was dissolved in distilled water and then dialyzed against distilled water in dialysis tubing having a molecular weight cut-off of 3,500.
(2) The supernatant from the Example 1 mixture was stored at 40° overnight, and a precipitate was then noted to have formed. The entirety of the contents of the container holding this was centrifuged at 12,000 rpm for 20 minutes. A pellet was recovered and was resuspended in 0.5 M NaCl with sonication. The resulting solution was dropwise precipitated in ten times the resuspension volume of ethanol at 4° C. The solution was stored overnight at 4° C. and a precipitate again formed. The solution and precipitate were centrifuged at 12,000 rpm for 20 minutes and a pellet was recovered. The pellet was dissolved in distilled water and dialyzed against distilled water in dialysis tubing having a 3,500 molecular weight cut-off.

Thereafter the dialyzed solutions from (1) and (2) above were pooled and lyophilized. Ninety mg. of *Haemophilus influenzae* type b polysaccharide antigen was obtained.

A solution of this antigen of 5.3 µg/ml concentration was prepared and subjected to Lowry assay for protein and found to contain 5 percent protein (wt/wt). The solution was also tested for carbohydrate by the phenol-sulfuric acid method and found to contain 36 percent (wt/wt). The solution was tested for activity by both the ELISA method and SDS-PAGE-immunoblot and found to have requisite activity.

### Example 3 -- Preparation of Affinity Column

Five mg. of lyophilized *Haemophilus influenzae* type b polysaccharide antigen was dissolved in 4.52 mL of distilled water and the pH was adjusted to 5-6 with HCl; 15.64 mg. of bovine serum albumen-hydrazine conjugate of pH 5-6 was then added, followed by mixing for three minutes.

2.6 µg of 1-ethyl-3-(3-dimethylaminopropyl) carbodiimide ("EDAC") was dissolved in 100 µL of distilled water. 50 µL of this solution was added to the antigen/BSA-hydrazine conjugate solution, followed by three minutes of mixing. The balance of the EDAC solution was then added to this mixture followed by two hours of mixing at room temperature. The pH was then adjusted to 8 with NaOH and mixed for one hour at room temperature, followed by storage overnight at 4° C.

The next day the pH of the stored mixture was adjusted to 7 with HCl and a portion was subjected to the ELISA test, confirming its activity.

2.12 mg. of the EDAC-treated antigen/BSA hydrazine conjugate was mixed with 2.4 g. of washed Spherilose^{™} gel and the resulting mixture was incubated at room temperature for two hours under top-to-bottom mixing conditions. 33.6 mg. of sodium cyanoborohydride was then dissolved in 480 µl of dissolved water and one-half of this solution was added to the antigen/BSA hydrazine conjugate/Spherilose™ gel mixture. The resulting mixture was incubated at room temperature for 3.5 hours under top-to-bottom mixing conditions. A coupled antigen/BSA hydrazine/Spherilose™ gel was separated and washed with 20 volumes of distilled water, followed by resuspension in 4.8 mL of 0.2 M Tris-HCl blocking buffer of pH 7. The remaining 240 µL of the above-described sodium cyanoborohydride solution was added to the suspension and this mixture was incubated at room temperature for one hour and then overnight at 4° C., under top-to-bottom mixing conditions throughout.

The coupled, blocked gel was separated and washed successively with 20 to 30 volumes of distilled water, triple strength phosphate buffered-saline of pH 7.2, standard strength phosphate-buffered saline of pH 9.2 and 3 M sodium thiocyanate in phosphate buffered saline of pH 7.5 to simulate a mock antibody purification and was packed onto an affinity column.

### Example 4 - Purification of H. influenzae type b Antibodies

To rabbit-α- *Haemophilus influenzae* type b serum, NaCl was added to a final concentration of 0.5 M NaCl and dissolved in the serum. The mixture was centrifuged at 5,000 XG for 20 minutes and filtered through cotton wool. Affinity gel from Example 3 was equilibrated with normal strength phosphate-buffered saline and the serum filtrate was applied to this gel four times. The gel was then washed with triple strength phosphate buffered saline, followed by normal strength phosphate buffered saline to remove unbound serum components.

Thereafter, the antibodies were eluted from the gel with 3 M sodium thiocyanate in phosphate buffered saline (pH = 7.5) followed by 3 M sodium thiocyanate in distilled water (pH 5 to 6). The recovered purified antibodies were dialyzed in normal strength phosphate buffered saline of pH 7.2.

### Example 5 -- ICT Assay for Haemophilus Influenzae type b

### A. Test Device Preparation

A test device comprising a hinged cardboard housing equipped with a window to allow the viewing of both the test results and control results was prepared as shown in Figure 1. The device has a recess into which is placed a preformed plastic swab well on its right-hand side (labeled 1 in the drawing) for receiving the sample-wetted swab. An overlabel show in Figure 1A is then placed over the entire right-hand side of the device. The overlabel has been equipped with two holes -- a lower one (marked B on Figure 1A) into which the saturated swab is to be inserted and an upper one (marked B on Figure 1A) toward which the swab will be pushed after insertion thereof into the hole B. The arrangement of the overlabel with its holes A and B, and the swab well cooperate to hold the swab in a proper position during the assay and to promote the expulsion of sorbed test sample liquid from the swab.

A preassembled test strip (marked B on Figure 1) described below, is inserted into the recess on the left-hand side (labeled 2 on Figure 1) and held in place by an adhesive applied to the bottom thereof. An overlabel shown in Figure 1B is placed atop the left-hand side. It has been equipped with a single hole (marked D in Figure 1B) which mates to the right-hand side hole A when the device is closed for performance of the assay.

The assembled device is stored in a sealed pouch with desiccant until it is used. Prior to sealing the pouch and storing, a lightly adhesive tape is placed on the outer edge of the right-hand half of the device.

### B. Test Strip Preparation and Construction

As Figure 1C shows, the test strip for the assay is comprised of a pad of sorbent material which has been impregnated with a conjugate of gold particles and affinity-purified rabbit anti-*Haemophilus influenzae* B antibodies. In use, this conjugate is rendered flowable by contact with the liquid test sample. The conjugate pad contacts a nitrocellulose pad onto which a capture line for sample that has reacted with the gold conjugate has been established by imbedding affinity-purified rabbit anti-*Haemophilus influenza* B antibodies therein. The nitrocellulose pad also includes a downstream control line established by striping the pad with goat anti-rabbit immunoglobin (IgG). After passing the nitrocellulose pad, the sample residue passes into an absorbent pad that serves as a reservoir for liquid.

The conjugate pad may be of non-woven polyester or extruded cellulose acetate. In preparing the pad for use in this assay, gold particles of 45 nm. diameter are conjugated, according to the method of DeMay, "The Preparation and Use of Gold Probes" in Immuno-chemistry; Modern Methods and Application (J. M. Polak and S. Van Norden, eds., Wright, Bristol, England, 1986) or any of various other known methods, to affinity purified anti*Haemophilus influenza* B antibodies. The affinity-purification is preferably achieved as described above. See also P. Tyssen, "Affinity chromatography of Immunoglobulins or Antibodies" contained in Practice and Theory of Enzyme Immunosassays (R.H. Burden and P.H. Van Knippedberg, eds., Elsevier, New York (1985). Any of various known methods of affinity purification may be substituted for the preferred method without departing from the present invention.

The gold conjugate particles are mixed with drying agent and embedded into a conjugate pad. The drying agent used is aqueous 5mM sodium tetraborate, pH 8.0, containing 1.0 percent bovine serum albumin, 0.1 percent Triton X-100, 2.0 percent Tween 20, 8.0 percent sucrose, and 0.02 percent sodium azide. The pad is heated sufficiently to remove all the liquid present and is stored in a low humidity environment pending assembly of the test device. These pads are especially chosen to hold the dry conjugate and to release it when wetted by sample.

The nitrocellulose pad is first treated by individually embedding affinity purified anti-*Haemophilus influenzae* b antibodies into a first portion of the pad. These antibodies act as the capture lines. A control line is established by striping goat anti-rabbit IgG on the surface of the pad. For those lines which are striped on the nitrocellulose pad, a solution consisting of 5mM sodium phosphate, pH 7.4, containing 5 percent methanol and 0.102 percent Intrawhite dye is used as a carrier fluid for the antibodies. The nitrocellulose pad is then desiccated at a temperature of 18-25° C. to promote permanent protein absorption thereto.

The absorbent pad used is of cellulosic material sold in commerce as Ahlstrom 243. It requires no special treatment. All the pads are assembled in the order shown in Figure 1C on an adhesive strip when the test device is put together for delivery to the customer.

### C. Immunoassay Procedure

In the conduct of the assay according to the invention, finished test devices having the swab well, the overlayers with holes and the test strip arranged as shown in the Figures are utilized. A swab fashioned from fibrous Dacron is briefly immersed in the urine sample and is then removed from the sample and immediately inserted, through the overlayer hole B on the right-hand side of the device, into the sample well of the test device. Two or three drops of "Reagent A", in this case a solution of 2.0 percent Tween 20, 0.05 percent sodium azide and 0.5 percent sodium dodecdyl sulfate in a 0.05 M sodium citrate-sodium phosphate buffer of pH 6.5 are added to the sample through the same hole. The adhesive strip on the edge of the right-hand side is peeled away and the device is then closed. The sample immediately contacts the conjugate pad and flows through the immunochromatographic strip. After 15 minutes, the test sample and control window are viewed and the results noted.

### D. Results of Sample Testing

A number of urine specimens of two types were analyzed in test devices as described above. The two types of urine samples evaluated were urine from patients without any pneumonia-type infection and urine containing *Haemophilus influenzae* b. All samples were tested in duplicate. The following chart summarizes the results of testing:

| **Sample** | ***Haemophilus influenzae B* Test Line** | **Control Line** |
|---|---|---|
| Urine from subjects without pneumonia | None | Positive |
| *Haemophilus influenzae b*-containing urine | Positive | Positive |

The results above were consistent for both a non-woven polyester conjugate pad and an extruded cellulose acetate conjugate pad. No differences were observed when either two or three drops of "Reagent A" were added.

### Example 6 -- Cross-Reactivity/Compatibility of Antigen-Specific Antibodies to H. Influenzae Type b

A commercial preparation of synthetic *H. influenza* type b sold under the label "ACT-HIB" by Pasteur-Merieux-Connaught Laboratories as *H. influenza* type b conjugate vaccine was injected into a rabbit and the rabbit was bled after the elapse of about 60 days.

The purified essentially protein-free capsular antigen as prepared in Example 2 was covalently conjugated to a hydrazine-BSA conjugate as shown in Example 3 and the antigen:hydrazine-BSA conjugate was in turn covalently coupled to the same affinity gel utilized in Example 3.

The rabbit serum containing raw polyclonal antibodies to *H. influenzae* type b was purified against the purified antigen:BSA-hydrazine affinity gel in the manner described in Example 4. The antigen-specific antibodies eluted from the gel were then utilized in compatibility and cross-reactivity tests, the results of which are graphed in Figures 2, 3 and 4 hereof.

### A. Compatibility Tests

The compatibility tests were performed using a modified ELISA method as follows:
96-well polystyrene microtiter plates from Dynex Technologies, Inc. were coated with 100 mcl. aliquots of various strains of *H. influenzae* cell suspension (0.5 - 0.7 x 108 cells/ml.). The plates were incubated at 37° C. for two hours and washed four times with PBS of pH 8.0 containing 0.02 percent Tween 20 ("PBST"). The microtiter wells were blocked with 200 mcl. of PBS of pH 7.2 containing BSA in a concentration of 1 mg./ml. for one hour at room temperature. The plates were then again washed four times with PBST.

The purified antigen-specific antibodies obtained from the rabbit immunized with commercial ACT-HIB as earlier described in this example were two-fold diluted through the plates starting at a concentration of 0.5 mcg./ml. and ending at 0.008 mcg./ml.

The first horizontal row on the plates was used as a control. Instead of antibody solution 100 mcl. of PBS was added to each well of this row. The plates were incubated for one hour at room temperature and then washed four times with PBST.

Thereafter 100 ml. of goat anti-rabbit IgG conjugated to horseradish peroxidase, diluted 1:6000 in PBST, was added to each well and the plates were incubated for 45 minutes at room temperature. After again washing with PBST, 100 mcl. of TMB Peroxidase Substrate System from KPL Laboratories, Gaithersburg, Maryland, was added to each well.

The reaction in each well was stopped with 50 mcl. of IN H₂SO₄ after three to five minutes of color development. The plates were counted at 450 nm wavelength in a spectrophotometric ELISA reader.

The various *H. influenzae* type b strains tested were products available from American Type Culture Collection under accession numbers #10211 (this being the strain utilized in Examples 1-5 hereof), #43335, #51654, and #43334. The results of the tests, which are graphed in Figure 2 hereof, show that the antigen-specific antibodies obtained by injecting a rabbit with ACT-HIB, bleeding the rabbit, and purifying the resultant antibody-containing rabbit serum with purified capsular antigen from ATCC #10211 according to the procedures of Examples 2 and 3 (designated as "[Hib-Ab]" in Figure 2), was most specific to and reactive with ATCC #10211, but still highly specific to and reactive with the capsular antigen of each of ATCC #43335, ATCC #51654 and #43334 at concentrations ranging from 0.063 mcg./ml. to 0.5 mcg./ml., when compared to the control. Moreover, using instrumental detection of the antigen-antibody reaction, the antigen-specific antibody of this invention produced discernible reactivity with antigen relative to the control at lower concentrations as low as 0.008 mg./mcl.

### B. Cross-Reactivity Tests

In these tests the antigen-specific purified *H. influenza* type b antibodies of this example were tested against other species of *H. influenza,* in two batches, following the test protocol described for the compatibility tests of Figure 2, using the same controls described for those tests.

For the first batch, Figure 3 is a graph comparing the reactivity of the antigen-specific antibodies of this invention with the antigen from ATCC #10211 to which they are specific, against antibodies of *H. influenzae* ("Hi" on the figure) types a, c, d and f. It demonstrates a lack of cross-reactivity with all of types a, c, d and f, as compared to high reactivity with and specificity for the type b *H. influenzae* antigen of ATCC #10211, at concentrations of 0.008 mcg./ml. to 0.063 mg./ml. A barely perceptible cross-reactivity with only *H. influenzae* type f is observable at concentrations of antibody slightly above 0.063 mcg./ml. but even at the highest concentrations of 0.5 mcg./ml. the reactivity with type f antigen is lower than that with ATCC #10211 type a at the lowest concentration of antibody of 0.008 mcg./ml. The slight cross-reactivity of type f with the antigen-specific antibody was adjudged too minor to be of concern.

For the second batch, Figure 4 is a graph comparing the reactivity of the purified antigen-specific antibodies of this invention with each of four non-typable *H. influenzae* species (NT1, NT2, NT3 and NT4) plus *H. parainfluenzae* as against the *H. influenzae* type b strain ATCC #10211. Figure 4 demonstrates lack of cross reactivity of the antigen-specific antibodies of the invention with all of the *H. influenzae* non-typable species 1, 2, 3 and 4 and very slight cross-reactivity with *H. parainfluenzae* at antibody concentrations of 0.125 mcg./ml. to 0.5 mcg./ml. This cross-reactivity, however, is of a lower order than the reactivity of the antibodies at a concentration of 0.008 mcg./ml. with type b *H. influenzae* strains ATCC #10211 and it was adjudged of negligible importance. Figure 4 also confirms the strong specificity of the antibodies of this invention for *H. influenza* type b capsular antigen.

Clearly, the purified antigen-specific antibodies of bacterial carbohydrate antigens can beneficially be utilized to detect the corresponding crude carbohydrate target antigen in any type of immunoassay and not just in those described herein. Equally clearly, substitution of these purified antigen-specific antibodies for raw polyclonal antibodies in previously described assays for the same target carbohydrate antigen will result in greater reliability, sensitivity and specificity of each such assay. Furthermore, it is believed, albeit not yet demonstrated, that substitution of these purified antigen-specific antibodies for monoclonal antibodies in assays described in the prior art will give results at least as good as and, it is expected, in many instances better and more reliable than those reported.

It is pointed out that the principles of this invention as herein disclosed lend themselves readily to a plethora of adaptations of, permutations of and combinations with assay techniques previously reported by others. Many of the steps disclosed herein can be accomplished using different reagents or conditions from those specifically disclosed. Other methods of purifying carbohydrate antigens to an essentially protein-free state can readily be devised. A vast array of literature, both patent and non-patent, discusses the design and use of reliable, one-time-use, disposable immunoassay test devices that could be substituted for the preferred ICT device described and recommended herein. It is not intended that the present invention should be limited with respect to substitutable assay devices, materials, ingredients or process steps except insofar as the following claims may so limit it.

## Claims

1. An immunochromatographic (ICT) assay for the detection of a target carbohydrate antigen component of *Haemophilus influenzae,* which comprises the steps of:
(a) contacting a sample of a fluid suspected of containing said target bacteria or their target carbohydrate antigen component with an immunochromatographic (ICT) device comprising a strip of a bibulous material, which strip has
(i) a zone in which has been embedded a conjugate of: (1) a labeling agent that displays a visible color change upon reaction of antibodies with their corresponding antigenic binding partner, and (2) purified antigen-specific antibodies to the target carbohydrate antigen component, said antibodies having been purified by passage over a chromatographic affinity column to which is conjugated through a spacer molecule the essentially protein-free purified target carbohydrate antigen component;
(ii) a second zone having bound thereto the same purified antigen specific antibodies in unconjugated form, which zone is equipped with a window for viewing color changes;
(b) allowing said sample to flow laterally along said test strip to said first zone;
(c) allowing said sample, together with said conjugate of affinity purified antibodies and label, to flow laterally along said test strip to said second zone; and
(d) within approximately 15 to 20 minutes from the commencement of step (a), observing through said window whether a line of color has appeared in said second zone, thereby indicating the presence in the sample of *Haemophilus influenzae,* or both, or whether no line of color has so appeared indicating the absence of *Haemophilus influenzae.*

2. The method of Claim 1 in which the target carbohydrate antigen component is a capsular carbohydrate antigen of *Haemophilus influenzae* type b bacteria, and the labeling agent is finely divided metallic gold.

3. A method according to Claim 1 in which the antigen-specific antibodies are present in a concentration of between 7.7 nanograms/mm² of surface area and 385 nanograms/mm² of surface area at each site of a test device at which antigen : antibody reaction is to occur.

## Patentansprüche

1. Immunchromatographischer (ICT) Test für den Nachweis einer Zielkomponente eines Kohlenhydratantigens von *Haemophilus influenzae,* welcher die Schritte umfasst:
(a) Inkontaktbringen einer Probe eines Fluids, das das Zielbakterium oder deren Zielkomponente eines Kohlenhydratantigens enthalten soll, mit einer immunchromatographischen (ICT) Vorrichtung, die einen Streifen eines saugfähigen Materials umfasst, wobei der Streifen aufweist:
(i) eine Zone, in der ein Konjugat eingebettet aus vorliegt: (1) einem Markierungsmittel, das auf Reaktion von Antikörpern mit deren entsprechenden antigenen Bindungspartnern eine sichtbare Farbänderung anzeigt, und (2) gereinigte antigenspezifische Antikörper, die gegen die Zielkomponente des Kohlenhydratantigens gerichtet sind, wobei die Antikörper durch eine Passage über eine chromatographische Affinitätssäule gereinigt wurden, an der durch ein Abstandshaltermolekül die wesentliche proteinfreie aufgereinigte Zielkomponente des Kohlenhydratantigens konjugiert vorliegt;
(ii) eine zweite Zone, die die gleichen gereinigten antigenspezifischen Antikörper in nicht konjugierter Form gebunden aufweist, wobei die Zone zum Betrachten von Farbänderungen mit einem Fenster ausgerüstet vorliegt;
(b) Ermöglichen, dass die Probe seitlich entlang des Teststreifens zu der ersten Zone fließen kann;
(c) Ermöglichen, dass die Probe zusammen mit dem Konjugat der affinitätsgereinigten Antikörper und dem Markierungsmittel seitlich entlang des Teststreifens zu der zweiten Zone fließen kann; und
(d) Beobachten durch das Fenster, ob innerhalb von ungefähr 15 bis 20 Minuten von Beginn des Schrittes (a) in der zweiten Zone eine Farblinie auftritt, wodurch die Gegenwart von *Haemophilus influenzae* in der Probe angezeigt wird, oder beides, oder ob keine Farblinie auftritt, was die Abwesenheit von *Haemophilus influenzae* anzeigt.

2. Verfahren nach Anspruch 1, wobei die Zielkomponente eines Kohlenhydratantigens ein Kapsel-Kohlenhydratantigen von *Haemophilus influenzae* Typ b Bakterien ist, und das Markierungsmittel fein, zerteiltes metallisches Gold.

3. Verfahren nach Anspruch 1, wobei die antigenspezifischen Antikörper in einer Konzentration zwischen 7,7 Nanogramm/mm² Oberflächenbereich und 385 Nanogramm/mm² Oberflächenbereich an jeder Stelle einer Testvorrichtung vorhanden sind, an der die Antigen : Antikörper-Reaktion auftreten soll.

## Revendications

1. Un procédé d'immuno chromatographie (ICT) pour la détection d'un composant antigène carbohydrate cible de *Haemophilus influenza* qui comprend les étapes de :
(a) mettre en contact un échantillon de fluide suspecté de contenir lesdites bactéries cibles ou leur composant antigène carbohydrate cible avec un dispositif d'immuno chromatographie (ICT) comprenant une bande de matériau absorbant, la bande comportant
(i) une zone dans laquelle a été incorporé un conjugué de : (1) un agent de marquage qui affiche un changement de couleur visible lors de la réaction des anticorps avec leur contrepartie antigénique de liaison correspondante, et (2) des anticorps purifiés spécifiques d'antigènes du composant antigéne carbohydrate cible, lesdits anticorps ayant été purifiés par passage sur une colonne de chromatographie par affinité à laquelle est conjuguée au moyen d'une molécule d'espacement le composant antigène carbohydrate purifié essentiellement exempt de protéine ;
(ii) une seconde zone à laquelle sont liés les mêmes anticorps spécifiques d'antigène purifiés sous forme non conjuguée, ladite zone étant munie d'une fenêtre pour visualiser les changements de couleur ;
(b) laisser s'écouler latéralement ledit échantillon le long da ladite bande test vers ladite première zone ;
(c) laisser ledit échantillon, avec lesdits conjugués d'anticorps purifiés par affinité et le marquer, s'écouler latéralement le long de ladite bande test vers la seconde zone ; et
(d) environ 15 à 20 minutes après le début de l'étape (a), observer par ladite fenêtre si une ligne de couleur est apparue dans ladite seconde zone, indiquant par ceci la présence dans l'échantillon de *Haemophilus influenza* ou les deux ou, lorsque aucune ligne de couleur apparaît, indiquant l'absence de *Haemophilus influenza.*

2. Le procédé de la revendication 1 dans lequel le composant antigène carbohydrate cible est un antigène carbohydrate capsulaire de bactérie *Haemophilus influenza* type b et l'agent de marquage est de l'or métallique finement divisé.

3. Un procédé selon la revendication 1 dans lequel les anticorps spécifiques d'antigène sont présents en une concentration comprise entre 7.7 nano grammes / mm2 de superficie et 385 nano grammes / mm2 de superficie sur chaque site d'une bande test sur laquelle la réaction antigène : anticorps se produit.
